Europäisches Patentamt

European Patent Office

Office européen des brevets

(19)

(11) Publication number: **0 283 231**
**A1**

## (12) EUROPEAN PATENT APPLICATION

(21) Application number: 88302206.3

(51) Int. Cl.⁴: **A61K 7/16**

(22) Date of filing: 14.03.88

(30) Priority: 16.03.87 GB 8706187

(43) Date of publication of application:
**21.09.88 Bulletin 88/38**

(84) Designated Contracting States:
**AT BE CH DE ES FR GB GR IT LI NL SE**

(71) Applicant: UNILEVER PLC
Unilever House Blackfriars P.O. Box 68
London EC4P 4BQ(GB)

(84) GB

(71) Applicant: UNILEVER NV
Burgemeester s'Jacobplein 1 P.O. Box 760
NL-3000 DK Rotterdam(NL)

(84) BE CH DE ES FR GR IT LI NL SE AT

(72) Inventor: **Bristow, Neil John**
**Flat 7 31 Cardiff Road**
**New Lambton Heights Newcastle NSW**
**2305(AU)**
Inventor: **Ingram, Geoffrey Stewart**
**The Cairngorms 4 Stanley Avenue**
**Bebington Wirral Merseyside L63 5QF(GB)**
Inventor: **Leon, Nicholas Hay**
**26 Mere Avenue**
**Raby Mere Wirral Merseyside L63 0NE(GB)**
Inventor: **Trevethan, Michael Albert**
**7 Warren Hey**
**Bebington Wirral L63 9LE(GB)**

(74) Representative: **Doucy, Robert Henry et al**
**Unilever PLC Patents Division P.O. Box 68**
**Unilever House**
**London EC4P 4BQ(GB)**

(54) **Dentifrice compositions.**

(57) The disclosure concerns a toothpaste having an aqueous humectant liquid phase, consisting at least in part of an aqueous solution of potassium acetate.

EP 0 283 231 A1

## DENTIFRICE COMPOSITIONS

This invention relates to dentifrice compositions, more particularly to those in the form of toothpastes, which are sometimes also referred to as dental creams.

Toothpastes generally comprise a suspension of a solid particulate material having abrasive properties in a liquid phase. They are usually dispensed from a collapsible tube or more recently also from a pump dispenser. It is necessary to include a humectant material in the liquid phase in order to prevent the toothpaste drying out in the nozzle of the tube or other dispenser. Several liquids have been proposed for use as a toothpaste humectant although the most commonly used ones in practice are glycerol, sorbitol syrup and propylene glycol.

It has now been discovered that an aqueous solution of potassium acetate has good humectant properties and can be used as a partial or complete replacement for conventional polyol humectants.

According to the present invention there is provided a toothpaste of which the liquid phase comprises humectant, characterised in that the humectant consists at least in part of an aqueous solution of potassium acetate, the amount of the potassium acetate being at least 2.5% by weight of the toothpaste.

The toothpaste of the invention will usually also comprise other conventional ingredients including abrasive cleaning or polishing agent, binder or thickening agent, surfactant and flavouring agent, with which ingredients potassium acetate is highly compatible.

While many abrasives agents have been proposed in the literature the silica and alumina abrasives which are in wide use are preferred. Silica abrasives are especially preferred and these may be either a silica gel, such as a silica xerogel, e.g. Syloid 63 available from W R Grace & Company, or a silica precipitate, e.g. Zeo 49, Zeodent 113 or Zeodent 119 all available from J M Huber Corporation. Others well known abrasives which may also be used include dicalcium phosphate dihydrate, insoluble sodium metaphosphate, calcium pyrophosphate, calcium carbonate and plastics materials. Abrasive agents are usually present in toothpastes in an amount of 5 to 75% by weight.

Likewise numerous binding or thickening agents have been indicated for use in toothpastes, preferred ones being sodium carboxymethylcellulose and xanthan gum. Others include natural gum binders such as gum tragacanth, gum karaya and gum arabic, Irish moss, alginates and carrageenans. Mixtures of binders may be used. The amount of binder included in a toothpaste is generally between 0.1 and 10% by weight.

It is usual to include a surfactant in a toothpaste and again the literature discloses a wide variety of suitable materials. Surfactants which have found wide use in practice are sodium lauryl sulphate, sodium dodecylbenzene sulphonate and sodium lauroylsarcosinate. Other anionic surfactants may be used as well as other types such as cationic, amphoteric and non-ionic surfactants. Surfactants are usually present in an amount of from 0.5 to 5% by weight of the toothpaste.

Together with the potassium acetate humectant, it will generally be desirable also to use another conventional humectant or mixture of humectants. However, a smaller amount of such conventional materials need be employed compared with the use of such materials as the sole humectant. Less potassium acetate may be used on a weight for weight basis than a conventional humectant which it replaces. Other known humectants which may be used in combination with potassium acetate include glycerol, sorbitol syrup, propylene glycol, lactitol, xylitol or hydrogenated starch hydrolysates. The total amount of humectant present will generally range from 10% to 85% by weight of the toothpaste. Apart from the humectant, water may also constitute part of the liquid phase of the toothpaste.

Flavours that are usually used in toothpastes are those based on oils of spearmint and peppermint. Examples of other flavouring materials used are menthol, clove, wintergreen, eucalyptus and aniseed. An amount of from 0.1% to 5% by weight of the toothpaste is a suitable amount of flavour to incorporate in a toothpaste.

The potassium acetate is incorporated in a toothpaste according to the invention in an amount of at least 2.5% by weight of the toothpaste. At amounts below 2.5% the humectancy benefit is minimal. Toothpastes can be formulated satisfactorily containing amounts of up to 40% potassium acetate but at high levels of usage flavouring problems are encountered. Preferably the potassium acetate is included in an amount of 2.5% to 25%, more preferably 4 to 15% by weight of the toothpaste.

The toothpaste of the invention may include a wide variety of optional ingredients with which potassium acetate is compatible. These include an anti-caries ingredient such as sodium fluoride or sodium monofluorophosphate or other effective fluorine-containing compound; an anti-plaque agent such as an antimicrobial compound for example chlorhexidine or a zinc salt (see EP-A-161 898); an anti-tartar ingredient such as a condensed phosphate e.g. an alkali metal pyrophosphate, hexametaphosphate or polyphosphate, (see US-A-4 515 772 and US-A-4 627 977); a sweetening agent such as saccharin; an opacifying agent, such as titanum dioxide; a preservative, such as formalin; a colouring agent; or pH controlling agent such as an acid, base or buffer, for example benzoic acid, to give an appropriate pH such as from about 4 to about 10.

For a fuller discussion of the formulation of toothpastes reference is made to Harry's Cosmeticology, Seventh Edition, 1982, Edited by J B Wilkinson and R J Moore, pages 609 to 617.

A particular advantage of the toothpaste of the invention is that the potassium acetate also imparts tooth desensitising properties to the toothpaste. Potassium nitrate is a potassium salt which is currently used in toothpastes as an agent for desensitising sensitive teeth. However, this salt has the disadvantage that it cannot be incorporated in sufficient amounts to be effective in toothpaste gels especially transparent gel toothpastes, which contain substantially less water than conventional opaque toothpastes (see Cosmetics & Toiletries, Vol.102, October 1987, page 81 for a discussion of toothpaste gels). However, potassium acetate because it is readily soluble in water is also satisfactory for the formulation of clear gel toothpastes with a tooth desensitising action. Clear gel toothpastes are characterised by a high humectant content (glycerol or sorbitol) and an abrasive or polishing agent having a refractive index similar to that of the liquid phase of the toothpaste. Silicas of satisfactory abrasive properties, such as the silica xerogels as described in US-A-3 538 230 (Pader et al), are particular useful for formulating transparent gel toothpastes. Precipitated silicas and sodium aluminosilicates have also been disclosed for use in gel toothpastes.

An amount of at least 2.5% potassium acetate based on the weight of the toothapste is considered necessary in order to obtain a satisfactory desensitising effect, and preferably at least 4% by weight is used.

US-A-3 897 548 (Katz) relates to an anti-plaque oral composition based on the combination of an antibacterial agent and a so-called "enamel conditioning agent" which is defined as any calcium ion chelating agent, acid or other agent capable of expanding (i.e. dissolving and/or loosening) the dental enamel crystal latice to facilitate uptake of the antibacterial agent, followed by a mechanism to provoke the subsequent reprecipitation and rehardening of the slightly dissolved tissue. The toothpaste of Example II of US-A-3 897 548 comprises as an ingredient potassium acetate in an amount of 2.0% by weight.

The following Examples illustrate the invention. Parts are by weight.

Example 1

The following is the formulation of an opaque toothpaste according to the invention.

| Ingredient | Parts |
| --- | --- |
| Alumina trihydrate | 50.00 |
| Sorbitol syrup (70% solution) | 22.00 |
| Potassium acetate | 5.00 |
| Sodium lauryl sulphate | 1.50 |
| Sodium carboxymethylcellulose | 1.00 |
| Sodium monofluorophosphate | 0.76 |
| Saccharin | 0.20 |
| Titanium dioxide | 1.00 |
| Flavour | 1.00 |
| Formalin | 0.04 |
| Benzoic acid | 0.10 |
| Water | 17.40 |

This toothpaste can be made by employing known mixing procedures. A suitable method is the following.

The sorbitol syrup, saccharin, sodium monofluorophosphate, potassium acetate, formalin and part of the water are mixed together to dissolve the solid ingredients. The alumina trihydrate, titanium dioxide and sodium carboxymethylcellulose are added in the next stage with stirring. The sodium lauryl sulphate is dissolved in the remaining water and then added to the mixture with stirring. Finally, the flavour is incorporated by mixing under vacuum.

Example 2

The following is an example of an opaque toothpaste according to the invention wherein potassium acetate is the sole humectant.

| Ingredient | Parts |
|---|---|
| Alumina trihydrate | 50.00 |
| Potassium acetate | 20.00 |
| Sodium lauryl sulphate | 1.50 |
| Sodium carboxymethylcellulose | 1.00 |
| Sodium monofluorophosphate | 0.76 |
| Saccharin | 0.20 |
| Titanium dioxide | 1.00 |
| Flavour | 1.00 |
| Benzoic acid | 0.10 |
| Citric acid | 0.20 |
| Water | 24.24 |

Example 3

The following is the formulation of a transparent gel toothpaste according to the invention.

| Ingredient | Parts |
|---|---|
| Silica xerogel | 7.00 |
| Precipitated silica | 6.50 |
| Sorbitol syrup (70% solution) | 66.00 |
| Glycerol | 10.00 |
| Potassium acetate | 5.00 |
| Polyethylene glycol 1500 | 3.00 |
| Sodium lauryl sulphate | 1.30 |
| Sodium dodecylbenzenesulphonate | 0.70 |
| Sodium carboxymethylcellulose | 0.50 |
| Sodium monofluorophosphate | 0.85 |
| Saccharin | 0.20 |
| Flavour | 1.00 |
| Magnesium sulphate | 0.50 |
| Trisodium phosphate | 0.065 |
| Sodium hydroxide | 0.10 |
| Colouring agent | 0.01 |
| Water | 2.275 |

5

Example 4

The following is the formulation of a translucent gel toothpaste according to the invention wherein potassium acetate is the sole humectant.

| Ingredient | Parts |
|---|---|
| Silica xerogel | 10.00 |
| Silica aerogel | 8.00 |
| Potassium acetate | 30.00 |
| Polyethylene glycol 1500 | 5.00 |
| Sodium lauryl sulphate | 1.50 |
| Sodium carboxymethylcellulose | 1.00 |
| Sodium monofluorophosphate | 0.80 |
| Saccharin | 0.20 |
| Flavour | 1.00 |
| Colour | 0.01 |
| Benzoic acid | 0.20 |
| Citric acid | 0.20 |
| Water | 42.09 |

While the above Examples 3 and 4 refer to the use of a silica xerogel other abrasive forms of silica may also be used including the abrasive silica precipitates, e.g. those sold under the name Syloblanc.

All the relative humidity (RH) data given below were obtained using a NOVASINA humidity sensor calibrated with lithium chloride (LiCl) (11.3% RH at 20°C), magnesium nitrate ($Mg(NO_3)_2$) (54.4% RH at 20°C), and barium chloride ($BaCl_2$) (90.5% RH at 20°C). The determinations were carried out at room temperature.

Experiments have been carried out comparing the humectant properties of potassium acetate with those of sorbitol syrup (70% solution). In one test a comparison was made between solutions A and B of composition given below.

Test Solution A - 5.0g potassium acetate, 22.0g sorbitol syrup (70% solution) and 17.5g water.
Control Solution B - 27.0g sorbitol syrup (70% solution) and 17.5g water.

The relative humidity data were as follows:-

| Solution | Relative Humidity (%) |
|---|---|
| Test Solution A | 89.0 |
| Control Solution B | 90.8 |

These data show that potassium acetate has good humectant properties and gives lower relative humidity values than an equal weight of sorbitol syrup.

The relative humidity of the toothpaste of Example 1 was compared to one in which the potassium acetate was replaced by sorbitol syrup, (referred to below as Comparative Example 1A). Data for the related formulation of Example 2 are also given.

The relative humidity data were as follows:-

| Toothpaste | Relative Humidity (%) |
|---|---|
| Example 1 | 83.6 |
| Comparative Example 1A | 88.1 |
| Example 2 | 59.1 |

The relative humidity of the toothpaste of Example 3 was compared to one from which the potassium acetate was omitted (referred to below as Comparative Example 3A).

The relative humidity data were as follows:-

| Toothpaste | Relative Humidity (%) |
|---|---|
| Example 3 | 64.5 |
| Comparative Example 3A | 69.8 |

The relative humidity of the toothpaste of Example 4 was 59.8%.

**Claims**

1. A toothpaste of which the liquid phase comprises humectant, characterised in that the humectant consists of at least in part of an aqueous solution of potassium acetate, the amount of the potassium acetate being at least 2.5% by weight of the toothpaste.

2. A toothpaste as claimed in claim 1, wherein the amount of the potassium acetate is from 2.5 to 25% by weight of the toothpaste.

3. A toothpaste as claimed in claim 2, wherein the amount of the potassium acetate is from 4 to 15% by weight of the toothpaste.

4. A toothpaste as claimed in any of the preceding claims, wherein the humectant comprises a mixture of the aqueous solution of potassium acetate and sorbitol syrup and/or glycerol.

5. A toothpaste as claimed in any of the preceding claims, wherein the toothpaste also comprises a silica abrasive.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,X | EP-A-0 161 898 (UNILEVER)<br>* Page 8, line 10 - page 10, line 2; page 24, lines 1-26 *<br>--- | 1-5 | A 61 K 7/16 |
| D,A | FR-A-2 224 162 (INDIANA UNIVERSITY FOUNDATION)<br>* Example 2 *<br>----- | 1-5 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 15-06-1988 | GOETZ G. |